(19) Europäisches Patentamt
European Patent Office
Office européen des brevets

(11) Publication number: **0 024 031 B2**

(12) **NEW EUROPEAN PATENT SPECIFICATION**

(45) Date of publication of the new patent specification: **20.05.87**

(51) Int. Cl.$^4$: **C 11 D 3/37,** C 11 D 1/90, C 11 D 1/75, C 11 D 3/48

(21) Application number: **80104605.3**

(22) Date of filing: **05.08.80**

(54) **Skin cleansing composition.**

(30) Priority: **13.08.79 US 65885**
**12.06.80 US 158737**

(43) Date of publication of application:
**18.02.81 Bulletin 81/07**

(45) Mention of the opposition decision:
**16.11.83 Bulletin 83/46**

(45) Publication of the grant of the patent:
**20.05.87 Bulletin 87/21**

(84) Designated Contracting States:
**AT BE CH DE FR GB IT LI LU NL SE**

(56) References cited:
**DE-A-1 467 931**
**DE-A-2 063 422**
**FR-A-2 406 439**
**GB-A-1 546 829**
**JP-A-54 004 312**
**US-A-3 849 348**
**US-A-4 048 301**

**Römpp's Chemielexikon Band 1, Seite 431**
**The Merck Index, 10th Addition, Referat 2057**
**CTFA Cosmetic Ingredient Dictionary (The Cosmetic, Toiletry and Fragrance Association, Inc., Washington) Second and Third Edition**

**The file contains technical information submitted after the application was filed and not included in this specification**

(73) Proprietor: **STERLING DRUG INC.**
**90 Park Avenue**
**New York New York (US)**

(72) Inventor: **Gorman, William George**
**25 Old Troy Road**
**East Greenbush New York (US)**
Inventor: **Popp, Karl Frederick**
**1775 Duck Pond Road**
**Schodack Landing New York (US)**

(74) Representative: **Baillie, Iain Cameron et al**
**c/o Ladas & Parry Isartorplatz 5**
**D-8000 München 2 (DE)**



Courier Press, Leamington Spa, England.

**Description**

The invention relates to cleansing compositions having improved sudsing ability for use in washing the skin.

The polyethylene glycol or polyoxyethylene esters of fatty acids are a known class of surfactants (W.B. Satkawski, S.K. Huang and R.L. Liss in Nonionic Surfactants, Martin J. Schick, Editor, Marcel Dekker, Inc. New York, 1967, pp. 142—174) of the nonionic type, members of which are listed by trade name (McCutcheon's Detergents & Emulsifiers, North American Edition, McCutcheon Division, MC Publishing Co., 175 Rock Road, Glen Rock, NJ 07452, 1977) and generic name (CTFA Cosmetic Ingredient Dictionary, Second Edition, The Cosmetic, Toiletry and Fragrance Association, Inc., 1133 Fifteenth Street, N.W., Washington, D.C. 20005, 1977).

The betain surfactants are also known and are of the amphoteric type (McCutcheon's Detergents & Emulsifiers, ibid.; CTFA Cosmetic Ingredient Dictionary, ibid.). Members of the class have antimicrobial properties as well as surfactant properties (Seymour S. Block, Disinfection, Sterilization and Preservation, ibid. pp. 348—360).

The amine oxide detergents are also known and are of the nonionic type (AROMOX Amine Oxides, Product Data Bulletin No. 74—21 of Armak Company, Box 1805, Chicago, Illinois, 60690, 1974; McCutcheon's Detergents and Emulsifiers, ibid., CTFA Cosmetic Ingredient Dictionary, ibid.).

There is a need for cleansing compositions having improved sudsing ability when compared with the compositions of the prior art.

According to the present invention a cleansing composition is provided, characterised by:

(1) from 0.75% to 30% by weight of one or more polyethylene glycol ester surfactants having the structural formulas.

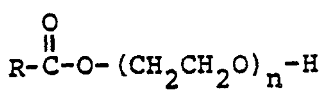

Formula V

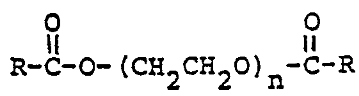

Formula VI

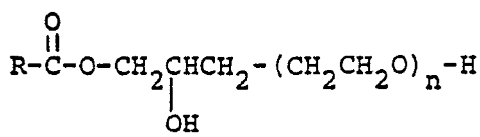

Formula VII

wherein R is alkyl or alkenyl having from 8 to 20 carbon atoms or lanolin and n is an integer from 8 to 200;

(2) from 0.5% to 30% by weight of one or more surfactants selected from

a) betains having the structural formulas

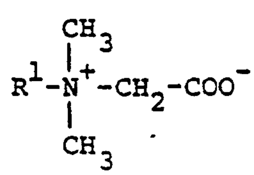

Formula VIII

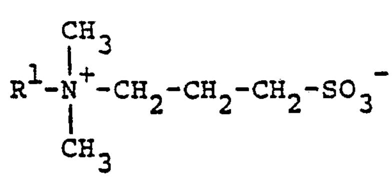

Formula IX

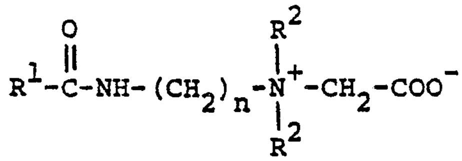

Formula X

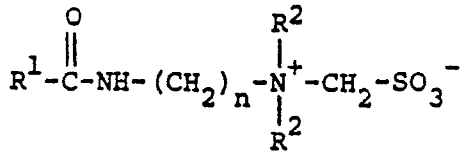

Formula XI

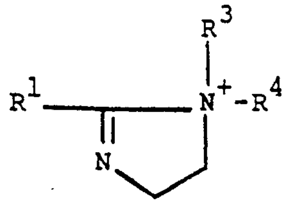

Formula XII

wherein $R^1$ is alkyl or alkenyl havving from 8 to 18 carbon atoms; $R^2$ is methyl, ethyl or 2-hydroxyethyl; $R^3$ is 2-hydroxyethyl or $CH_2COO^-$; $R^4$ is $CH_2COO^-$ or $CH_2CH_2$—O—$CH_2COO^-$; an n is 2 or 3; and

b) amine oxides having the structural formula

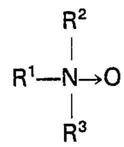

Formula XII

wherein $R^1$ taken alone is methyl, ethyl or 2-hydroxyethyl; $R^2$ taken alone is methyl, ethyl or 2-hydroxyethyl; $R^1$ and $R^2$ taken together are morpholino; $R^3$ is alkyl having from 8 to 18 carbon atoms or $R^4$ $CONH(CH_2)_3$ wherein $R^4$ is alkyl having from 8 to 18 carbon atoms, and wherein 2-hydroxyethyl can be condensed with from 1 to 200 units of ethylene oxide;

(3) from 0.01% to 10% by weight of an antimicrobial agent which is:

a) a compound having the structural Formula I

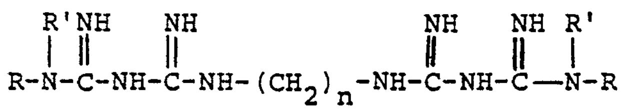

Formula I

wherein R taken alone is phenyl substituted by alkyl, alkoxy, nitro or halo, p-(2,2-dichlorocyclopropyl)phenyl, alkyl having from 6 to 16 carbon atoms, cycloalkyl or polycyclic alkyl having more than 6 carbon atoms or lower-alkyl-cycloalkyl or cycloalkyl-lower-alkyl having from 1 to 4 carbon atoms in lower alkyl; R′ taken alone is hydrogen; R and R′ taken together are 3-azabicyclo(3,2,2)nonyl; and n is an integer from 3 to 9; or a pharmaceutically acceptable salt thereof; or

b) a compound having the structural Formula IV

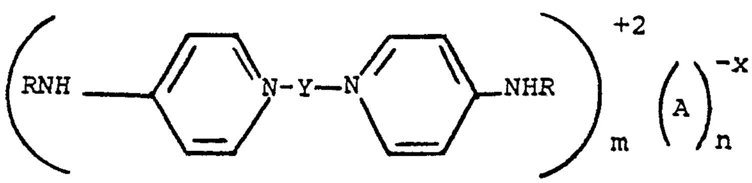

Formula IV

wherein R is an alkyl group containing from 6 to 18 carbon atoms, a cycloalkyl group containing from 5 to 7 carbon atoms, benzyl, benzyl substituted by one or two substituents selected from halogen, hydroxy, lower-alkyl, lower-alkoxy, nitro, cyano and trifluoromethyl or phenyl substituted by methylenedioxy or one or two substituents selected from halogen, lower-alkyl, lower-alkoxy, nitro, cyano and trifluoromethyl;

Y is an alkylene group containing from 4 to 18 carbon atoms and separating the two 4-(R—NH)-1-pyridinyl groups by from 4 to 18 carbon atoms;

A is a pharmaceutically acceptable anion;

m is 1 or 3;

n is 1 or 2;

x is 1, 2 or 3; and

(m) (2) = (n) (x); and

(4) water, aqueous ethyl alcohol, aqueous isopropyl alcohol or an aqueous ethyl alcohol-isopropyl alcohol mixture to make 100% by weight.

The ingredients of formulae (V) to (XIII) of the cleansing compositions are generally and, in most

instances, particularly described by the prior art cited above. The compositions are prepared by conventional methods and in addition may also include adjuncts, for example, emollients, lubricants, stabilizers, dyes, perfumes and preservatives. A suitable acid or base may also be added for pH adjustment.

Antimicrobial aryl bisbiguanides (U.S.—A—2,684,924; U.S.—A—4,053,636) including chlorhexidine (The Merck Index, Ninth Edition, 1976, monograph 2060) and chlorhexidine digluconate salt (U.S.—A—2,990,425) are known. U.S.—A—3,855,140 describes polyoxyethylenepolyoxypropylene block copolymer cleansing compositions of certain chlorhexidine salts including chlorhexidine digluconate salt. Antimicrobial alkyl bisbiguanidines (U.S.—A—3,468,898) including alexidine (The Merck Index, ibid., monograph 224) and aqueous compositions thereof with "a compatible surfactant or surfactant mixture selected from the cationic non-ionic and amphoteric surfactants" (BE—A—862,808) and cycloalkyl bisbiguanides (U.S.—A—4,022,834) are also known.

Antimicrobial bispyridines, and compositions thereof "with any compatible, pharmaceutically acceptable surfactant, preferably, a non-ionic surfactant, such as the polyoxyethylene polyoxypropylene copolymers . . . amine oxides, such as stearyl dimethyl amine oxide . . . or with mixtures of these" are described by GB—A—1,533,952.

JP—A—54 043 12 (Derwent Reference 37652) describes polyethylene glycol distearate and/or dipalmitate — amphoteric surface active agent (e.g., imidazoline) shampoo compositions, but does not suggest that such a composition include an antimicrobial agent.

GB—A—1,546,829 generally describes liquid washing and cleansing agents and requires the presence of "about 7 to 40% by weight of non-ionic surface active agent, especially a $C_{10}$—$C_{20}$-alkylpolyglycol ether and/or $C_6$—$C_{20}$-alkylphenolpolyglycol ether". The use of an antimicrobial agent in such a composition is again not suggested.

The inventive antimicrobial compositions have improved sudsing ability as compared to these of the prior art, e.g., those of U.S.—A—3,855,140.

One can reduce the number of microbes on living skin by applying to the skin an antimicrobially effective amount of any one of the above described antimicrobial cleansing composition.

The preferred amount of antimicrobial agent in the composition for skin application is from 0.01% to 10% by weight of the composition. In spite of the presence of the antimicrobial agent, a preservative may be necessary to prevent growth of microorganisms in the antimicrobial compositions. The adjuncts used in the antimicrobial compositions should be pharmaceutically acceptable.

Particularly preferred bisbiguanides are the following compounds of Formula I.

| Compound of Formula | R | R′ | n |
|---|---|---|---|
| Ia | *p*-$ClC_6H_5$ | H | 6 |
| Ib | $Cl_2CCH_2CHC_6H_5$ | H | 6 |
| Ic | $CH_3(CH_2)_3CH(CH_2CH_3)CH_2$ | H | 6 |
| Id | $CH_3(CH_2)_6$ | H | 6 |

The compound of Formula Ia is chlorhexidine (The Merck Index, ibid., monograph 2060). The compound of Formula Ic is alexidine (ibid., monograph 224). The digluconate salt is a particularly preferred bisbiguanide salt (chlorhexidine gluconate).

The following bispyridines of Formula IV are particularly preferred.

| Compound of Formula | R | Y | A | m | n | x |
|---|---|---|---|---|---|---|
| IVa | $CH_3(CH_2)_6$ | (CH.$(CH_2)_{12}$ | Cl or Br | 1 | 2 | 1 |
| IVb | $CH_3(CH_2)_7$ | $(CH_2)_{10}$ | Cl or Br | 1 | 2 | 1 |
| IVc | $CH_3(CH_2)_3CHCH_2$<br>$CH_2CH_3$ | $(CH_2)_{12}$ | Cl or Br | 1 | 2 | 1 |

Although any polyethylene glycol ester surfactant of Formula V—VII and any betain surfactant of Formula VIII—XII and any amine oxide surfactant of Formula XIII can be used in carrying out this invention, specific members of each class may show advantages in specific application areas. Some that have been commercialized only recently are not listed in the current editions of CTFA Cosmetic Ingredient Dictionary (ibid.) or McCutcheon's Detergents & Emulsifiers (ibid.).

Particularly preferred polyethylene glycol ester surfactants are tabulated below.

| Generic Name | CTFA Cosmetic Ingredient Dictionary Page |
|---|---|
| PEG—150 Laurate | 219 |
| PEG—150 Distearate | 211 |
| PEG—78 Glyceryl Cocate (Cf. PEG—7 Glyceryl Cocoate) | 212 |
| PEG—30 Glyceryl Cocoate (Cf. PEG—7 Glyceryl Cocoate) | 212 |

Particularly preferred betaine surfactants are tabulated below.

| Generic Name | Type | CTFA Cosmetic Ingredient Dictionary Page |
|---|---|---|
| Coco-betaine | Formula VIII | 67 |
| Coco-sultaine | Formula IX | 68 |
| Cocamidopropyl Betaine | Formula X | 65 |
| Cocamidopropyl Sultaine | Formula XI | 66 |
| Amphoteric-1 | Formula XII | 24 |

Particularly preferred amine oxide surfactants are tabulated below.

| Generic Name | CTFA Cosmetic Ingredient Dictionary Page |
|---|---|
| Myristamine Oxide | 179 |
| Coco-morpholine Oxide | 67 |
| Cocamidopropylamine Oxide | 65 |
| Dihydroxyethyl Cocamine Oxide | 95 |

Compatibility of the antimicrobial agents of Formula Id, Formula IVa (A=Br), Formula IVb (A = Br) and Formula IVc (A = Br) with a prototype vehicle was tested by a serial dilution test against *Staphylococcus aureus ATCC 6538.* Volumes of two milliliters were used. Dilutions were in tryptosephosphate broth. Incubation was for 16 to 18 hours at 37°C. Growth was verified with 2,3,5-triphenyltetrazolium chloride. The inoculum was $1.8 \times 10^5$ viable cells per tube. There follows the formula of the prototype vehicle.

| Ingredient | Percent by Weight |
|---|---|
| PEG—150 | 12.0 |
| Isopropyl Alcohol* | 3.22 |
| Cocamidopropyl Betaine | 3.00 |
| Laneth-16** | 1.00 |
| Edetate Disodium*** | 0.500 |
| PEG—150 Distearate | 0.500 |
| PEG—2M**** | 0.100 |
| Perfume | 0.100 |
| FD&C Blue No. 1 | 0.000800 |
| FD&C Yellow No. 5 | 0.000320 |
| Gluconic Acid or Sodium Hydroxide to make | pH about 5.5 |
| Purified Water to make | 100.0 |

* To make 4% by volume
** Polyethyleneglycol ether of lanolin-alcohol with an average EO 30 value of 16
*** Disodium salat of ethylene diamine tetraacetic acid
**** H—(—$OCH_2CH_2$)$_n$—OH n = 2000

The following results were obtained.

| Added Vehicle µg/ml) | Minimum Inhibitory Concentration (MIC) (µg/ml) of Compound of Formula Id⁺ | IIa | IVa° | IVb° | IVc° |
|---|---|---|---|---|---|
| 0 | 0.25 | 0.5 | 0.39 | 0.5 | 0.25 |
| 15.6 | 0.25 | 0.5 | 0.195 | 0.5 | 0.25 |
| 31.2 | 0.25 | 0.5 | 0.39 | 0.5 | 0.25 |
| 62.5 | 0.25 | 0.5 | 0.39 | 0.5 | 0.25 |
| 125.0 | 0.25 | 0.5 | 0.10 | 0.5 | 0.125 |
| 250.0 | 0.25 | 0.5 | 0.10 | 0.25 | 0.0625 |
| 500.0 | 0.25 | 0.5 | 0.10 | 0.125 | 0.125 |
| 1000.0 | 0.25 | 1.0 | 0.195 | 0.125 | 0.125 |
| 2000.0 | 0.50 | 1.0 | 0.10 | 0.125 | 0.125 |
| 4000.0 | 0.50 | 1.95 | 0.195 | 0.25 | 0.25 |
| 8000.0 | 0.50 | 1.95 | 0.10 | 0.25 | 0.25 |
| 16000.0 | * | * | * | * | * |
| 32000.0 | ** | ** | ** | ** | ** |
| 64000.0 | ** | ** | ** | ** | ** |

⁺ Expressed as free base
° Expressed as cation
* MIC of vehicle alone
** No growth at this concentraton of vehicle

It was concluded that each of the five antimicrobial agents tested was compatible with the prototype vehicle.

## Examples

The following examples still more particularly describe the compositions of the invention.

### Example 1

| Ingredient | Percent by Weight |
|---|---|
| Chlorhexidine gluconate | 4.00 |
| PEG—78 Glyceryl Cocate | 10.0 |
| Amphoteric-1 | 5.00 |
| Laneth-16 | 1.00 |
| Benzyl Alcohol | 1.00 |
| Acetamide MEA | 0.750 |
| PEG—30 Glyceryl Cocoate | 0.500 |
| Color | 0.00100 |
| Perfume | 0.0500 |
| Gluconic Acid to make pH about 5.5, about | 2.19 |
| Purified Water to make | 100.0 |

### Example 2

| Ingredient | Percent by Weight |
|---|---|
| Compound of Formula IVb (A = Cl) | 2.00 |
| PEG—150 Laurate | 12.0 |
| Ispropyl Alcohol | 3.22 |
| Cocamidopropyl Betaine | 5.00 |
| Laneth-16 | 1.00 |
| Edetate Disodium | 0.500 |
| PEG—150 Distearate | 0.500 |
| PEG—2M | 0.100 |
| Perfume | 0.100 |
| Color | 0.00100 |
| Sodium Hydroxide to make pH about 5.5 | 0.023 |
| Purified Water to make | 100.00 |

Example 3

| Ingredient | Percent by Weight |
| --- | --- |
| Compound of Formula IVb (A = Cl) | 2.00 |
| PEG—78 Glyceryl Cocoate | 10.0 |
| Dihydroxyethyl Cocoamine Oxide | 5.00 |
| Citric Acid | 0.384 |
| Sodium Hydroxide to make pH about 5.5 | — |
| Purified Water to make | 100.00 |

Example 4

| Ingredient | Percent by Weight |
| --- | --- |
| Chlorhexidine gluconate | 4.00 |
| Amphoteric-1 | 2.50 |
| Cocamidopropylamine Oxide | 2.00 |
| PEG—78 Glyceryl Cocoate | 10.0 |
| PEG—30 Glyceryl Cocoate | 0.500 |
| Lanet-16 | 1.00 |
| Acetamide MEA | 0.750 |
| Color | 0.00100 |
| Perfume | 0.0500 |
| Gluconic Acid or Sodium Hydroxide to make pH about 5.5 | — |
| Acetic Acid | 0.500 |
| Benzyl Alcohol | 1.00 |
| Purified Water to make | 100.00 |

Example 5

| Ingredient | Percent by Weight |
|---|---|
| Chlorhexidine Gluconate | 4.00 |
| Dihydroxyethyl Cocoamine Oxide | 3.00 |
| PEG—150 Distearate | 0.750 |
| Laneth-16 | 1.00 |
| Benzyl Alcohol | 2.00 |
| Perfume | 0.100 |
| Color | 0.00100 |
| Gluconic Acid or Sodium Hydroxide to make pH about 5.5 | — |
| Purified Water to make | 100.00 |

The composition of Example 2 was tested for antimicrobial effect on human hands using a gloved-hand model (R. N. Michaud, M.B. McGrath and W.A. Goss, Journal of Clinical Microbiology, vol. 3, 1976, pp. 406—413). Twelve haands were randomly assigned for washes with the composition of Example 2, and twelve hands were randomly assigned for washes with a corresponding composition differing only by absence of the antimicrobial agent (control). Each hand was washed four times within six hours with a minimum of one hour between washes using five milliters of each compostion for each wash. During each wash the opposite hand of each person was protected with a surgical glove.

Microbiological samples for quantitative determination of each resident bacteria were obtained from each hand immediately prior to the first (Wash 1 Pre), after a glove-wearing period of one hour subsequent to the first (Wash 1 $T_1$), second (Wash 2 $T_1$) and fourth (Wash 4 $T_1$) washes, and after a non-gloveing-wearing period of twenty hours after the fourth wash (Wash 4 $T_{20}$). Total bacterial counts per hand were expressed as logarithms to the base 10. The hand-degerming effect after one wash was evaluated as the bacterial reduction (Wash 1 Pre-Wash 1 $T_1$), abbreviated ($W_1$Pre-$W_1T_1$). Cumulative effects after two washes and four washes were evaluated as the bacterial reductions (Wash 1 Pre-Wash 2 $T_1$), abbreviated ($W_1$Pre-$W_2T_1$), and (Wash 1 Pre-wash 4 $T_1$), abbreviated ($W_1$Pre-$W_4T_1$). Persistent effect was evaluated as the bacterial reduction (Wash 1 Pre-Wash 4 $T_{20}$) abbreviated ($W_1$Pre-$W_4T_{20}$).

Microbiological samples were taken by extracting each gloved hand with sampling fluid (100 ml. of 0.1% Triton X—100 in 0.074M phosphate buffer of pH 7.8±0.1). An aliquot (10 ml.) of each extract was mixed with chilled neutralizer (10 ml. of 0.0003M potassium dihydrogen phosphate buffer of pH 7.2 containing 2% Tamol N Micro brand of sodium salt of condensed naphthalene sulfonic acid anionic dispersant). Decmila dilutions ($10^{-1}$, $10^{-2}$, $10^{-3}$) of the neutralized aliquot were prepared in 0.0003M $KH_2PO_4$ buffer, pH 7.2, and were plated in triplicate with trypticase soy agar using the pour-plate technique. Plates were incubated aerobically at 30±2°C. for 48—72 hours. A total colony count for each plate was determined using an automated colony counter. Average colony counts were used to estimate the total number of bacteria recovered per hand.

The following results were obtained

| | Mean (n = 12) $Log_{10}$ | Number of Bacteria Recovered per Hand |
|---|---|---|
| Sample | Control | Example 2 |
| Wash 1 Pre | 6.372 | 6.165 |
| Wash 1 $T_1$ | 6.537 | 5.770 |
| Wash 2 $T_1$ | 6.316 | 5.030 |
| Wash 4 $T_1$ | 5.978 | 4.296 |
| Wash 4 $T_{20}$ | 5.846 | 4.867 |

| Difference | Mean (n = 12) $Log_{10}$ Control | Bacterial Reductions Per Hand Example 2 |
|---|---|---|
| $W_1Pre-W_1T_1$ | −0.166 | 0.394 |
| $W_1Pre-W_2T_1$ | 0.055 | 1.135 |
| $W_1Pre-W_4T_1$ | 0.393 | 1.869 |
| $W_1Pre-W_4T_{20}$ | 0.525 | 1.297 |

The foregoing mean $\log_{10}$ bacterial reductions were significantly greater ($P \leqslant 0.01$) for the hands treated with the composition of Example 2 than for the hands treated with the control composition.

**Claims for the Contracting States: BE CH DE FR GB IT LI LU NL SE**

1. Cleansing composition characterized by:

(1) from 0.75% to 30% by weight of one or more polyethylene glycol ester surfactants having the structural Formulas V, VI or VII

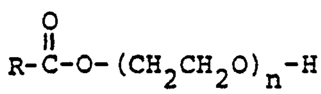

Formula V

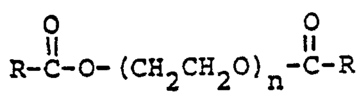

Formula VI

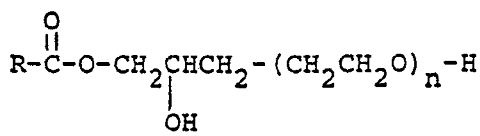

Formula VII

wherein R is alkyl or alkenyl havving from 8 to 20 carbon atoms or lanolin and n is an integer from 8 to 200;

(2) from 0.5% to 30% by weight of one or more surfactants selected from a) betaines having the structural Formulas VIII, IX, X, XI or XII

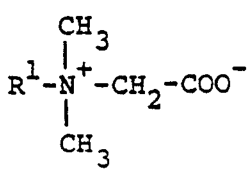

Formula VIII

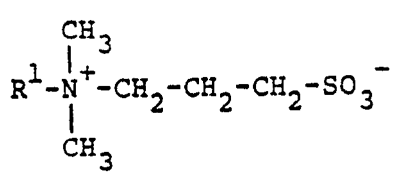

Formula IX

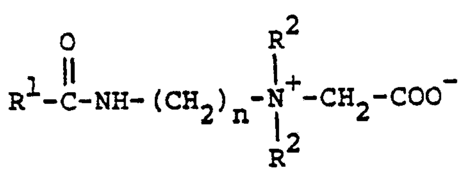

Formula X

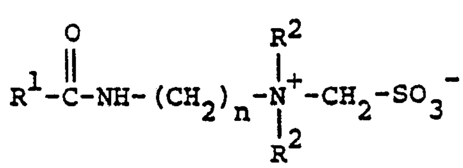

Formula XI

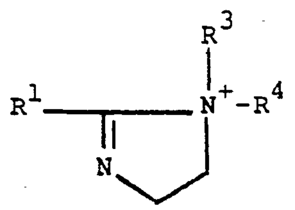

Formula XII

wherein $R^1$ is alkyl or alkenyl having from 8 to 18 carbon atoms; $R^2$ is methyl, ethyl or 2-hydroxyethyl; $R^3$ is 2-hydroxyethyl or $CH_2COO^-$; $R^4$ is $CH_2COO^-$ or $CH_2CH_2—O—CH_2COO^-$; and n is 2 or 3; and

b) amine oxides having the structural Formula XIII

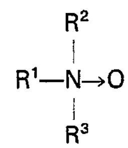

Formula XIII

wherein $R^1$ taken alone is methyl, ethyl or 2-hydroxyethyl; $R^2$ taken alone is methyl, ethyl or 2-hydroxyethyl; $R^1$ and $R^2$ taken together are morpholino; $R^3$ is alkyl having from 8 to 18 carbon atoms or $R^4CONH(CH_2)_3$ wherein $R^4$ is alkyl having from 8 to 18 carbon atoms, and wherein hydroxyethyl can be condensed with from 1 to 200 units of ethylene oxide;

(3) from 0.01% to 10% by weight of an antimicrobial agent which is:

a) a compound having the structural Formula I

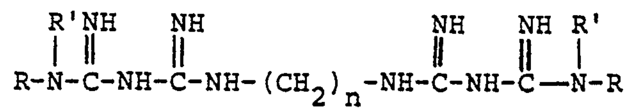

Formula I

wherein R taken alone is phenyl substituted by alkyl, alkoxy, nitro or halo, p-(2,2-dichlorocyclopropyl)phenyl, alkyl having from 6 to 16 carbon atoms, cycloalkyl or polycyclic alkyl having more than 6 carbon atoms or lower-alkyl-cycloalkyl or cycloalkyl-lower-alkyl having from 1 to 4 carbons in lower alkyl; R′ taken alone is hydrogen; R and R′ taken together are 3-azabicyclo(3,2,2)nonyl; and n is an integer from 3 to 9; or a pharmaceutically acceptable salt thereof; or

b) a compound having the structural Formula IV

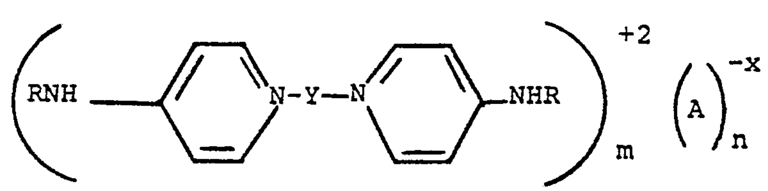

Formula IV

wherein R is an alkyl group containing from 6 to 18 carbon atoms, a cycloalkyl group containing from 5 to 7 carbon atoms, benzyl, benzyl substituted by one or two substituents selected from halogen, hydroxy, lower-alkyl, lower-alkoxy, nitro, cyano and trifluoromethyl or phenyl substituted by methylenedioxy or one or two substituents selected from halogen, lower-alkyl, lower-alkoxy, nitro, cyano and trifluoromethyl;

Y is an alkylene group containing from 4 to 18 carbon atoms and separating the two 4-(R—NH)-1-pyridinyl groups by from 4 to 18 carbon atoms;

A is a pharmaceutically acceptable anion;

m is 1 or 3;

n is 1 or 2;

x is 1, 2 or 3; and

(m) (2) = (n) (x); and

(4) water, aqueous ethyl alcohol, aqueous isopropyl alcohol or an aqueous ethyl alcohol-isopropyl alcohol mixture to make 100% by weight.

2. A composition according to claim 1, characterized by the fact that the antimicrobial agent is chlorhexidine or a pharmaceutically acceptable salt thereof.

3. A composition according to claim 2, characterized by the fact that the pharmaceutically acceptable salt is the digluconate salt.

4. A composition according to claim 1, characterized by the fact that in the structural Formula IV of the antimicrobial agent R is $CH_3(CH_2)_7$, Y is $(CH_2)_{10}$, A is Cl or Br, m is 1, n is 2 and x is 1.

5. A composition according to any one of the preceding claims, for reducing the number of microbes on living skin.

**Claims for the Contracting State: AT**

1. Cleansing composition characterized by:

(1) from 0.75% to 30% by weight of one or more polyethylene glycol ester surfactants having the structural Formulas V, VI or VII

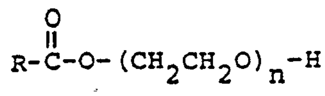

Formula V

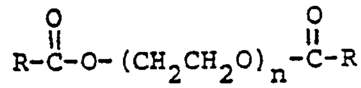

Formula VI

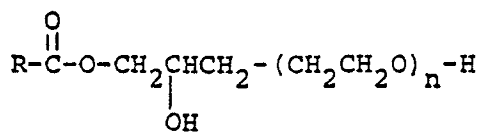

Formula VII

wherein R is alkyl or alkenyl having from 8 to 20 carbon atoms or lanolin and n is an integer from 8 to 200;

(2) from 0.5% to 30% by weight of one or more surfactants selected from a) betaines having the structural Formulas VIII, IX, X, XI or XII

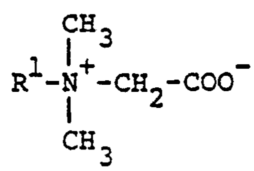

Formula VIII

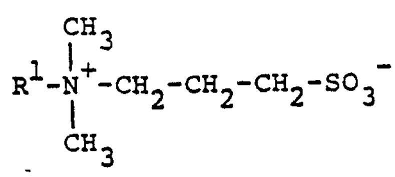

Formula IX

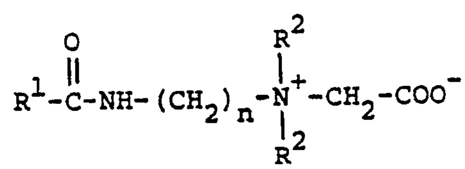

Formula X

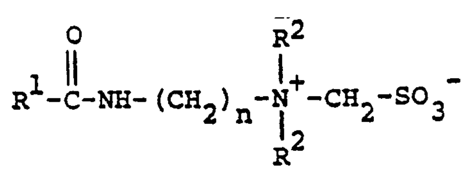

Formula XI

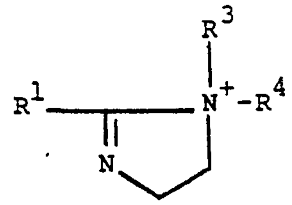

Formula XII

wherein $R^1$ is alkyl or alkenyl having from 8 to 18 carbon atoms; $R^2$ is methyl, ethyl or 2-hydroxyethyl; $R^3$ is 2-hydroxyethyl or $CH_2COO^-$; $R^4$ is $CH_2COO^-$ or $CH_2CH_2—O—CH_2COO^-$; and n is 2 or 3; and

b) amine oxides having the structural Formula XIII

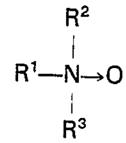

Formula XIII

wherein $R^1$ taken alone is methyl, ethyl or 2-hydroxyethyl; $R^2$ taken alone is methyl, ethyl or 2-hydroxyethyl; $R^1$ and $R^2$ taken together are morpholino; $R^3$ is alkyl having from 8 to 18 carbon atoms or $R^4CONH(CH_2)_3$ wherein $R^4$ is alkyl having from 8 to 18 carbon atoms, and wherein hydroxyethyl can be condensed with from 1 to 200 units of ethylene oxide;

(3) from 0.01% to 10% by weight of an antimicrobial agent which is:

a) a compound having the structural Formula I

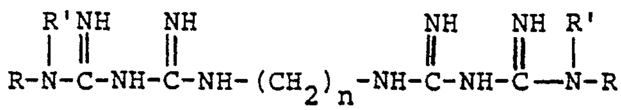

Formula I

wherein R taken alone is phenyl substituted by alkyl, alkoxy, nitro or halo, p-(2,2-dichlorocyclopropyl)phenyl, alkyl having from 6 to 16 carbon atoms, cycloalkyl or polycyclic alkyl having more than 6 carbon atoms or lower-alkyl-cycloalkyl or cycloalkyl-lower-alkyl having from 1 to 4 carbons in lower alkyl; R′ taken alone is hydrogen; R and R′ taken together are 3-azabicyclo(3,2,2)nonyl; and n is an integer from 3 to 9; or a pharmaceutically acceptable salt thereof; or

b) a compound having the structural Formula IV

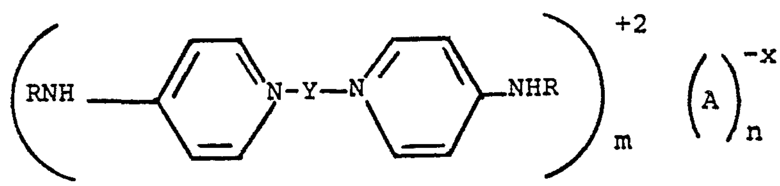

Formula IV

wherein R is an alkyl group containing from 6 to 18 carbon atoms, a cycloalkyl group containing from 5 to 7 carbon atoms, benzyl, benzyl substituted by one or two substituents selected from halogen, hydroxy, lower-alkyl, lower-alkoxy, nitro, cyano and trifluoromethyl or phenyl substituted by methylenedioxy or one or two substituents selected from halogen, lower-alkyl, lower-alkoxy, nitro, cyano and trifluoromethyl;

Y is an alkylene group containing from 4 to 18 carbon atoms and separating the two 4-(R—NH)-1-pyridinyl groups by from 4 to 18 carbon atoms;

A is a pharmaceutically acceptable anion;

m is 1 or 3;

n is 1 or 2;

x is 1, 2 or 3; and

(m) (2) = (n) (x); and

(4) water, aqueous ethyl alcohol, aqueous isopropyl alcohol or an aqueous ethyl alcohol-isopropyl alcohol mixture to make 100% by weight.

2. A composition according to claim 1, characterized by the fact that the antimicrobial agent is chlorhexidine or a pharmaceutically acceptable salt thereof.

3. A composition according to claim 2, characterized by the fact that the pharmaceutically acceptable salt is the digluconate salt.

4. A composition according to claim 1, characterized by the fact that in the structural Formula IV of the antimicrobial agent R is $CH_3(CH_2)_7$, Y is $(CH_2)_{10}$, A is Cl or Br, m is 1, n is 2 and x is 1.

**Patentansprüche für die Vertragsstaaten: BE CH LI DE FR GB IT LU NL SE**

1. Reinigungszusammensetzung, gekennzeichnet durch:

(1) 0,75 bis 30 Gew.-% eines oder mehrerer Polyäthylenglycolestertenside der Strukturformeln V, VI oder VII

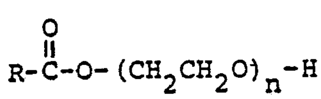

Formel V

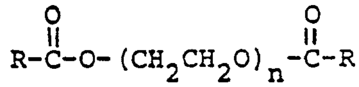

Formel VI

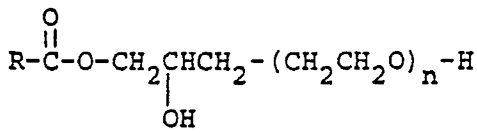

Formel VII

worin R Alkyl oder Alkenyl mit 8 bis 20 Kohlenstoffatomen oder Lanolin ist und n eine ganze Zahl von 8 bis 200 ist;

(2) 0,5 bis 30 Gew.-% eines oder mehrerer Tenside, ausgewählt aus

a) Betainen der Strukturformeln VIII, IX, X, XI oder XII

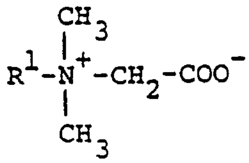

Formel VIII

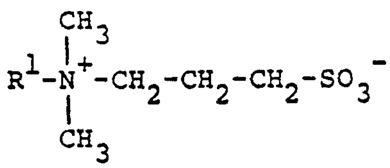

Formel IX

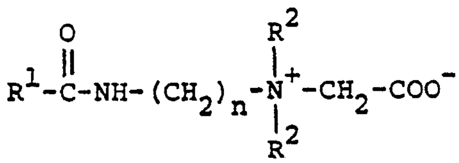

Formel X

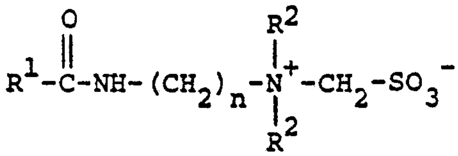

Formel XI

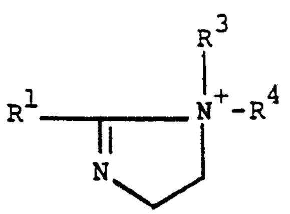

Formel XII

worin $R^1$ Alkyl oder Alkenyl mit 8 bis 18 Kohlenstoffatomen ist; $R^2$ Methyl, Äthyl oder 2-Hydroxyäthyl; $R^3$ 2-Hydroxyäthyl oder $CH_2COO^-$; $R^4$ $CH_2COO^-$ oder $CH_2CH_2—O—CH_2COO^-$; und n 2 oder 3 ist; und

b) Aminoxiden der Strukturformel XIII

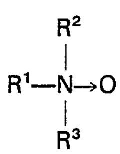

Formel XIII

worin $R^1$ für sich allein Methyl, Äthyl oder 2-Hydroxyäthyl; $R^2$ für sich allein Methyl, Äthyl oder 2-Hydroxyäthyl; $R^1$ und $R^2$ zusammengenommen Morpholino; $R^3$ Alkyl mit 8 bis 18 Kohlenstoffatomen oder $R^4CONH(CH_2)_3$ bedeutet, worin $R^4$ Alkyl mit 8 bis 18 Kohlenstoffatomen ist, und worin Hydroxyäthyl mit 1 bis 200 Äthylenoxideinheiten kondensiert sein kann;

(3) 0,01 Gew.-% bis 10 Gew.-% eines antimikrobiellen Mittels, welches ist:

a) eine Verbindung der Strukturformel I

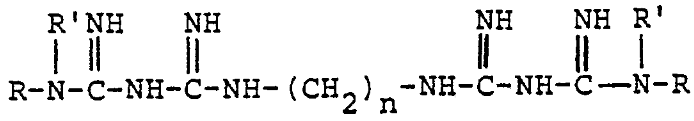

Formel I

worin R für sich allein durch Alkyl, Alkoxy, Nitro oder Halogen substituiertes Phenyl, p-(2,2-Dichlorcyclopropyl)phenyl, Alkyl mit 6 bis 16 Kohlenstoffatomen, Cycloalkyl oder polycyclisches Alkyl mit mehr als 6 Kohlenstoffatomen oder niedr.-Alkyl-Cycloalkyl oder Cycloalkyl-niedr.-Alkyl mit 1 bis 4 Kohlenstoffatomen im niedr.-Alkyl ist; R' für sich allein Wasserstoff ist; R und R' zusammengenommen 3-Azabicyclo(3,2,2)nonyl ist; und n eine ganze Zahl von 3 bis 9 ist; oder ein pharmazeutisch annehmbares Salz davon; oder

b) eine Verbindung der Strukturformel IV

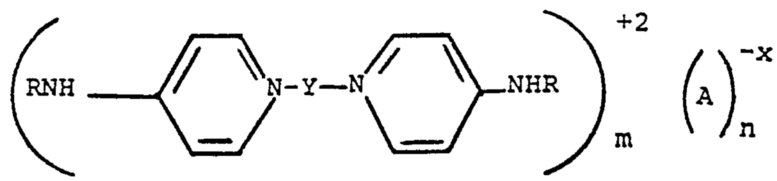

Formel IV

worin R eine Alkylgruppe mit 6 bis 18 Kohlenstoffatomen, eine Cycloalkylgruppe mit 5 bis 7 Kohlenstoffatomen, Benzyl, durch einen oder zwei aus Halogen, Hydroxy, niedr.-Alkyl, niedr.-Alkoxy, Nitro, Cyano und Trifluormethyl ausgewählte Substituenten substituiertes Benzyl oder durch Methylendioxy oder einen oder zwei aus Halogen, niedr.-Alkyl, niedr.-Alkoxy, Nitro, Cyano und Trifluormethyl ausgewählte Substituenten substituiertes Phenyl ist;

Y eine Alkylengruppe mit 4 bis 18 Kohlenstoffatomen, wleche die zwei 4-(R—NH)-1-Pyridinylgruppen durch 4 bis 18 Kohlenstoffatome voneinander trennt, ist;

A ein pharmazeutisch annehmbares Anion ist;

m 1 oder 3 ist;

n 1 oder 2 ist;

x 1, 2 oder 3 ist; und

(m) (2) = (n) (x); und

(4) Wasser, wäßrigen Äthylalkohol, wäßrigen Isopropylalkohol oder ein wäßriges Äthylalkohol-Isopropylalkoholgemisch, ergänzend auf 100 Gew.-%.

2. Zusammensetzung nach Anspruch 1, dadurch gekennzeichnet, daß das antimikrobielle Mittel Chlorhexidin oder ein pharmazeutisch annehmbares Salz davon ist.

3. Zusammensetzung nach Anspruch 2, dadurch gekennzeichnet, daß das pharmazeutisch annehmbare Salz das Digluconatsalz ist.

4. Zusammensetzung nach Anspruch 1, dadurch gekennzeichnet, daß in der Strukturformel IV des antimikrobiellen Mittels R $CH_3(CH_2)_7$ ist, Y $(CH_2)_{10}$ ist, A Cl oder Br ist, m 1 ist, n 2 ist und x 1 ist.

5. Zusammensetzung nach einem der vorhergehenden Ansprüche zur Verminderung der Anzahl von Mikroben auf lebender Haut.

**Patentansprüche für den Vertragsstaat: AT**

1. Reinigungszusammensetzung, gekennzeichnet durch:

(1) 0,75 bis 30 Gew.-% eines oder mehrerer Polyäthylenglycolestertenside der Strukturformeln V, VI oder VII

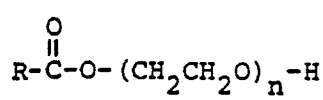

Formel V

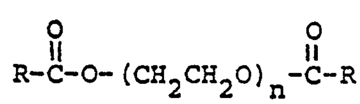

Formel VI

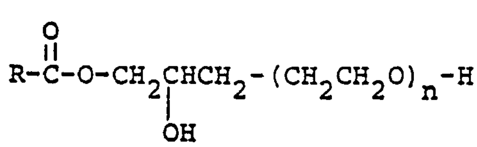

Formel VII

worin R Alkyl oder Alkenyl mit 8 bis 20 Kohlenstoffatomen oder Lanolin ist und n eine ganze Zahl von 8 bis 200 ist;

(2) 0,5 bis 30 Gew.-% eines oder mehrerer Tenside, ausgewählt aus

a) Betainen der Strukturformeln VIII, IX, X, XI oder XII

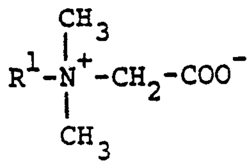

Formel VIII

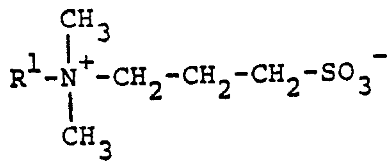

Formel IX

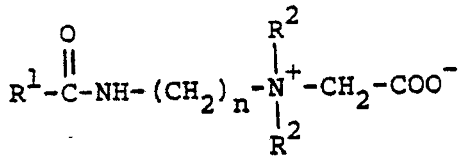

Formel X

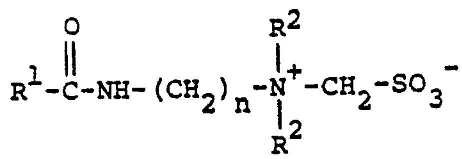

Formel XI

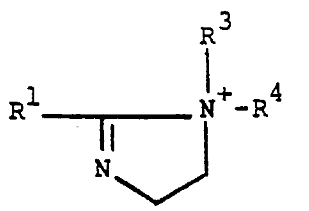

Formel XII

worin $R^1$ Alkyl oder Alkenyl mit 8 bis 18 Kohlenstoffatomen ist; $R^2$ Methyl, Äthyl oder 2-Hydroxyäthyl; $R^3$ 2-Hydroxyäthyl oder $CH_2COO^-$; $R^4$ $CH_2COO^-$ oder $CH_2CH_2—O—CH_2COO^-$; und n 2 oder 3 ist; und

b) Aminoxiden der Strukturformel XIII

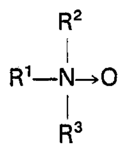

Formel XIII

worin $R^1$ für sich allein Methyl, Äthyl oder 2-Hydroxyäthyl; $R^2$ für sich allein Methyl, Äthyl oder 2-Hydroxyäthyl; $R^1$ und $R^2$ zusammengenommen Morpholino; $R^3$ Alkyl mit 8 bis 18 Kohlenstoffatomen oder $R^4CONH(CH_2)_3$ bedeutet, worin $R^4$ Alkyl mit 8 bis 18 Kohlenstoffatomen ist, und worin Hydroxyäthyl mit 1 bis 200 Äthylenoxideinheiten kondensiert sein kann;

(3) 0,01 Gew.-% bis 10 Gew.-% eines antimikrobiellen Mittels, welches ist:

a) eine Verbindung der Strukturformel I

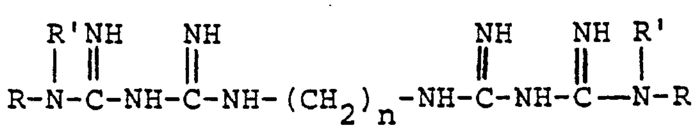

Formel I

worin R für sich allein durch Alkyl, Alkoxy, Nitro oder Halogen substituiertes Phenyl, p-(2,2-Dichlorcyclopropyl)phenyl, Alkyl mit 6 bis 16 Kohlenstoffatomen, Cycloalkyl oder polycyclisches Alkyl mit

mehr als 6 Kohlenstoffatomen oder niedr.-Alkyl-Cycloalkyl oder Cycloalkyl-niedr.-Alkyl mit 1 bis 4 Kohlenstoffatomen im niedr.-Alkyl ist; R' für sich allein Wasserstoff ist; R und R' zusammengenommen 3-Azabicyclo(3,2,2)nonyl ist; und n eine ganze Zahl von 3 bis 9 ist; oder ein pharmazeutisch annehmbares Salz davon; oder

b) eine Verbindung der Strukturformel IV

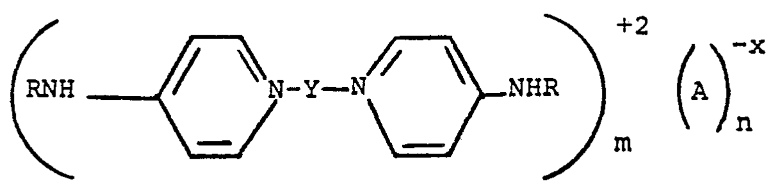

Formel IV

worin R eine Alkylgruppe mit 6 bis 18 Kohlenstoffatomen, eine Cycloalkylgruppe mit 5 bis 7 Kohlenstoffatomen, Benzyl, durch einen oder zwei aus Halogen, Hydroxy, niedr.-Alkyl, niedr.-Alkoxy, Nitro, Cyano und Trifluormethyl ausgewählte Substituenten substituiertes Benzyl oder durch Methylendioxy oder einen oder zwei aus Halogen, niedr.-Alkyl, niedr.-Alkoxy, Nitro, Cyano und Trifluormethyl ausgewählte Substituenten substituiertes Phenyl ist;

Y eine Alkylengruppe mit 4 bis 18 Kohlenstoffatomen, welche die zwei 4-(R—NH)-1-Pyridinylgruppen durch 4 bis 18 Kohlenstoffatome voneinander trennt, ist;

A ein pharmazeutisch annehmbares Anion ist;

m 1 oder 3 ist;

n 1 oder 2 ist;

x 1, 2 oder 3 ist; und

(m) (2) = (n) (x); und

(4) Wasser, wäßrigen Äthylalkohol, wäßrigen Isopropylalkohol oder ein wäßriges Äthylalkohol-Isopropylalkoholgemisch, ergänzend auf 100 Gew.-%.

2. Zusammensetzung nach Anspruch 1, dadurch gekennzeichnet, daß das antimikrobielle Mittel Chlorhexidin oder ein pharmazeutisch annehmbares Salz davon ist.

3. Zusammensetzung nach Anspruch 2, dadurch gekennzeichnet, daß das pharmazeutisch annehmbare Salz das Digluconatsalz ist.

4. Zuysammensetzung nach Anspruch 1, dadurch gekennzeichnet, daß in der Strukturformel IV des antimikrobiellen Mittels R $CH_3(CH_2)_7$ ist, Y $(CH_2)_{10}$ ist, A Cl oder Br ist, m 1 ist, n 2 ist und x 1 ist.

**Revendications pour les Etats contractants: BE CH LI DE FR GB IT LU NL SE**

1. Composition de nettoyage, caractérisée par:

(1) de 0,75% à 30% en poids d'un ou plusieurs surfactifs du type ester de polyéthylène-glycol ayant les Formules développées V, VI ou VII

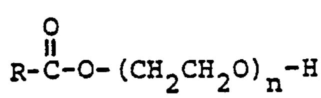

Formule V

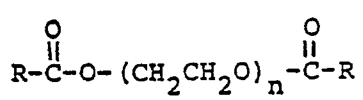

Formule VI

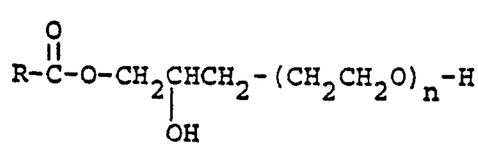

Formule VII

dans lesquelles R est un groupe alkyle ou alcényle ayant 8 à 20 atomes de carbone ou la lanoline et n est un nombre entier de 8 à 200;

(2) de 0,5% à 30% en poids d'un ou plusieurs surfactifs choisis parmi:

a) des bétaïnes ayant les Formules développées VIII, IX, X, XI ou XII

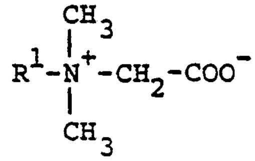

Formule VIII

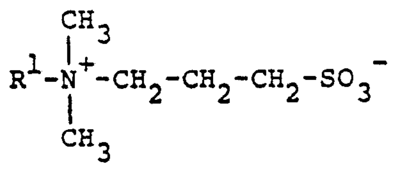

Formule IX

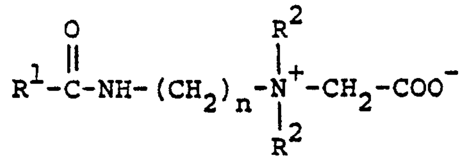

Formule X

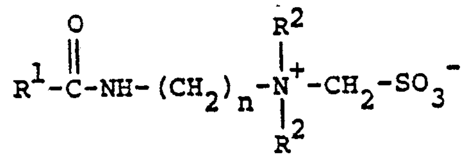

Formule XI

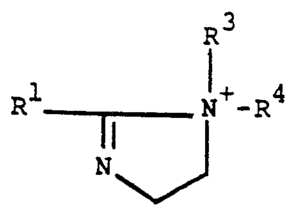

Formule XII

dans lesquelles $R^1$ est un groupe alkyle ou alcényle ayant 8 à 18 atomes de carbone, $R^2$ est un groupe méthyle, éthyle ou 2-hydroxyéthyle; $R^3$ est le groupe 2-hydroxyéthyle ou $CH_2COO^-$; représente $CH_2COO^-$ ou $CH_2CH_2—O—CH_2COO^-$; et n est égal à 2 ou 3; et

b) les oxydes d'amine ayant la Formule développée XIII

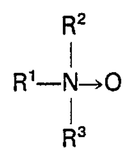

Formule XIII

dans laquelle $R^1$, considéré seul, est un groupe méthyle, éthyle ou 2-hydroxyéthyle; $R^2$, considéré seul, est un groupe méthyle, éthyle ou 2-hydroxyéthyle; $R^1$ et $R^2$, pris ensemble, représentent le groupe morpholino; $R^3$ est un groupe alkyle ayant 8 à 18 atomes de carbone ou $R^4CONH(CH_2)_3$ où $R^4$ est un groupe alkyle ayant 8 à 18 atomes de carbone, et où le radical hydroxyéthyle peut être condensé avec 1 à 200 motifs d'oxyde d'éthylène;

(3) de 0,01% à 10% en poids d'un agent antimicrobien qui est:

a) un composé ayant la Formule développée I

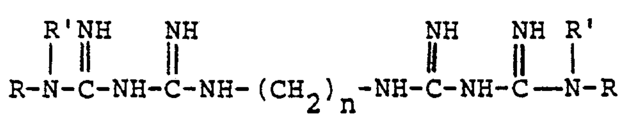

Formule I

dans laquelle R considéré seul est un groupe phényle substitué par un groupe alkyle, alkoxy, nitro ou halogéno, p-(2,2-dichlorocyclopropyl)phényle, alkyle ayant 6 à 16 atomes de carbone, cycloalkyle ou alkyle polycyclique ayant plus de 6 atomes de carbone ou (alkyl inférieur) cycloalkyle ou cycloalkyl (alkyle inférieur) ayant 1 à 4 atomes de carbone dans la portion alkyle inférieur; R' considéré seul est l'hydrogène; R et R' pris ensemble représentent le groupe 3-azabicyclo(3,2,2)nonyle; et n est un nombre entier de 3 à 9; ou un sel pharmaceutiquement acceptable de ce composé';

b) un composé ayant la Formule développée IV

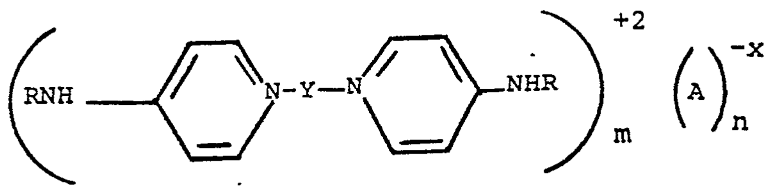

Formule IV

dans laquelle R est un groupe alkyle contenant 6 à 18 atomes de carbone, un groupe cycloalkyle contenant 5 à 7 atomes de carbone, benzyle, benzyle substitué par un ou deux substituants choisis parmi les halogènes, les groupes hydroxy, alkyle inférieur, alkoxy inférieur, nitro, cyano et trifluorométhyle ou phényle substitué par un radical méthylènedioxy ou un ou deux substituants choisis parmi les halogènes, les groupes alkyle inférieur, alkoxy inférieur, nitro, cyano et trifluorométhyle;

Y est un groupe alkylène contenant 4 à 18 atomes de carbone et séparant les deux groupes 4-(R—NH)-1-pyridinyle par 4 à 18 atomes de carbone;

A est un anion pharmaceutiquement acceptable;

m est égal à 1 ou 3;

n est égal à 1 ou 2;

x est 1, 2 ou 3; et

(m) (2) = (n) (x); et

(4) de l'eau, de l'alcool éthylique aqueux, de l'alcool isopropylique aqueux, ou un mélange auqueux d'alcool éthylique et d'alcool isopropylique pour constituer 100% en poids.

2. Composition selon la revendicaation 1, caractérisée par le fait que l'agent antimicrobien est la chlorhexidine ou un sel pharmaceutiquement acceptable de cette dernière.

3. Composition selon la revendication 2, caractérisée par le fait que le sel pharmaceutiquement acceptable est le digluconate.

4. Composition selon la revendication 1, caractérisé par le fait que dans la formule développée IV de l'agent antimicrobien, R est $CH_3(CH_2)_7$, Y est $(CH_2)_{10}$, A est Cl ou Br, m est égal à 1, n est égal à 2 et x est égal à 1.

5. Composition selon l'une quelconque des revendications précédentes, pour réduire le nombre de microbes sur de la peau vivante.

**Revendications pour l'Etat contractant: AT**

1. Composition de nettoyage, caractérisée par:

(1) de 0,75% à 30% en poids d'un ou plusieurs surfactifs du type ester de polyéthylène-glycol ayant les Formules développées V, VI ou VII

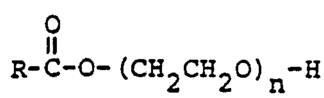

Formule V

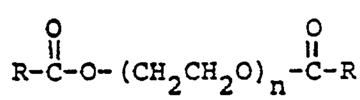

Formule VI

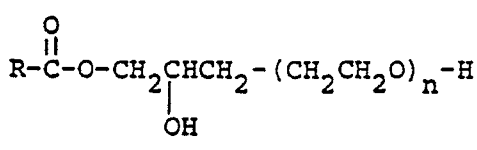

Formule VII

dans lesquelles R est un groupe alkyle ou alcényle ayant 8 à 20 atomes de carbone ou la lanoline et n est un nombre entier de 8 à 200;

(2) de 0,5% à 30% en poids d'un ou plusieurs surfactifs choisis parmi:

a) des bétaïnes ayant les Formules développées VIII, IX, X, XI ou XII

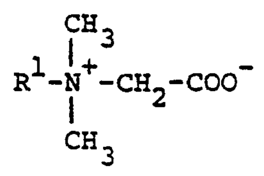

Formule VIII

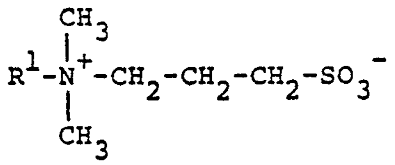

Formule IX

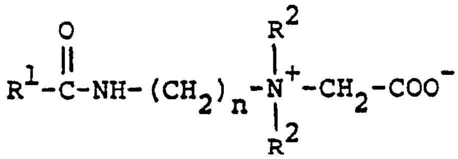

Formule X

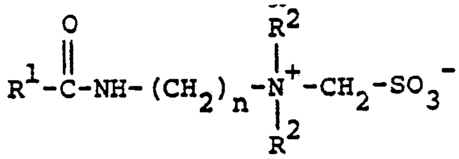

Formule XI

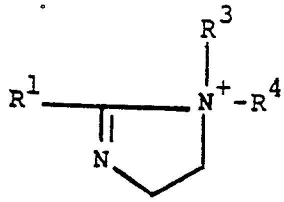

Formule XII

dans lesquelles $R^1$ est un groupe alkyle ou alcényle ayant 8 à 18 atomes de carbone, $R^2$ est un groupe méthyle, éthyle ou 2-hydroxyéthyle; $R^3$ est le groupe 2-hydroxyéthyle ou $CH_2COO^-$; représente $CH_2COO^-$ ou $CH_2CH_2$—O—$CH_2COO^-$; et n est égal à 2 ou 3; et

b) les oxydes d'amine ayant la Formule développée XIII

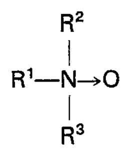

Formule XIII

dans laquelle $R^1$, considéré seul, est un groupe méthyle, éthyle ou 2-hydroxyéthyle; $R^2$, considéré seul, est un groupe méthyle, éthyle ou 2-hydroxyéthyle; $R^1$ et $R^2$, pris ensemble, représentent le groupe morpholino; $R^3$ est un groupe alkyle ayant 8 à 18 atomes de carbone ou $R^4CONH(CH_2)_3$ où $R^4$ est un groupe alkyle ayant 8 à 18 atomes de carbone, et où le radical hydroxyéthyle peut être condensé avec 1 à 200 motifs d'oxyde d'éthylène;

(3) de 0,01% à 10% en poids d'un agent antimicrobien qui est:

a) un composé ayant la Formule développée I

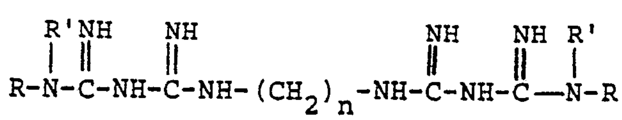

Formule I

dans laquelle R considéré seul est un groupe phényle substitué par un groupe alkyle, alkoxy, nitro ou halogéno, p-(2,2-dichlorocyclopropyl)phényle, alkyle ayant 6 à 16 atomes de carbone, cycloalkyle ou alkyle polycyclique ayant plus de 6 atomes de carbone ou (alkyl inférieur) cycloalkyle ou cycloalkyl (alkyle inférieur) ayant 1 à 4 atomes de carbone dans la portion alkyle R' considéré seul est l'hydrogène; R et R' pris ensemble représentent le groupe 3-azabicyclo(3,2,2)nonyle; et n est un nombre entier de 3 à 9; ou un sel pharmaceutiquement acceptable de ce compose';

b) un composé ayant la Formule développée IV

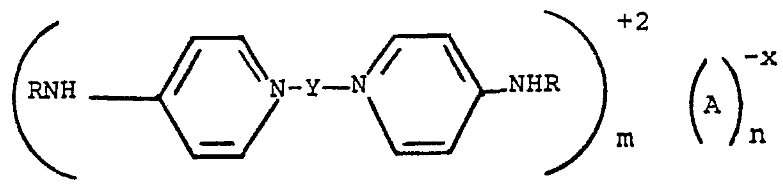

Formule IV

dans laquelle R est un groupe alkyle contenant 6 à 18 atomes de carbone,.un groupe cycloalkyle contenant 5 à 7 atomes de carbone, benzyle, benzyle substitué par un ou deux substituants choisis parmi les halogènes, les groupes hydroxy, alkyle inférieur, alkoxy inférieur, nitro, cyano et trifluorométhyle ou phényle substitué par un radical méthylènedioxy ou un ou deux substituants choisis parmi les halogènes, les groupes alkyle inférieur, alkoxy inférieur, nitro, cyano et trifluorométhyle;

Y est un groupe alkylène contenant 4 à 18 atomes de carbone et séparant les deux groupes 4-(R—NH)-1-pyridinyle par 4 à 18 atomes de carbone;

A est un anion pharmaceutiquement acceptable;

m est égal à 1 ou 3;

n est égal à 1 ou 2;

x est 1, 2 ou 3; et

(m) (2) = (n) (x); et

(4) de l'eau, de l'alcool éthylique aqueux, de l'alcool isopropylique aqueux, ou un mélange auqueux d'alcool éthylique et d'alcool isopropylique pour constituer 100% en poids.

2. Composition selon la revendicaation 1, caractérisée par le fait que l'agent antimicrobien est la chlorhexidine ou un sel pharmaceutiquement acceptable de cette dernière.

3. Composition selon la revendication 2, caractérisée par le fait que le sel pharmaceutiquement acceptable est le digluconate.

4. Composition selon la revendication 1, caractérisé par le fait que dans la formule développée IV de l'agent antimicrobien, R est $CH_3(CH_2)_7$, Y est $(CH_2)_{10}$, A est Cl ou Br, m est égal à 1, n est égal à 2 et x est égal à 1.